Europäisches Patentamt

European Patent Office

Office européen des brevets

(19)

(11) Publication number: **0 382 395**
**A1**

## (12) EUROPEAN PATENT APPLICATION

(21) Application number: **90300992.6**

(22) Date of filing: **31.01.90**

(51) Int. Cl.⁵: **A61F 2/36**

(30) Priority: **08.02.89 US 308176**
**10.01.90 US 457999**

(43) Date of publication of application:
**16.08.90 Bulletin 90/33**

(84) Designated Contracting States:
**BE CH DE DK FR GB IT LI LU NL SE**

(71) Applicant: **SMITH & NEPHEW RICHARDS INC.**
**1450 Brooks Road**
**Memphis, Tennessee 38116-1892(US)**

(72) Inventor: **Brumfield, David L.**
**1200 Hall Road**
**Nesbit, Mississippi 38651(US)**
Inventor: **Durham, A. Glenn**
**4458 Dunn**
**Memphis, Tennessee 38117(US)**
Inventor: **Russell, Thomas A.**
**6438 Massey Estate Cove**
**Memphis, Tennessee 38119(US)**
Inventor: **Small, Laura C.**
**6369 Arbor Creek, No. 105**
**Memphis, Tennessee 38115(US)**

(74) Representative: **Harrison, Philippa Dinah et al**
**A. A. THORNTON & CO Northumberland**
**House 303-306 High Holborn**
**London WC1V 7LE(GB)**

(54) **Modular system for femoral fixation.**

(57) A modular apparatus is provided which combines the advantages of a total hip prosthesis with the superior fixation of an interlocking intramedullary nail. The apparatus includes an intramedullary rod, a separate prosthetic, generally spherical surface member (18) for forming the exterior of the femoral head, and means for securing the rod to the prosthetic surface. The prosthetic surface member (18) may be a total hip prosthesis or a femoral head resurfacing or replacement member.

Fig.2.

## MODULAR SYSTEM FOR FEMORAL FIXATION

The present invention relates to femoral fixation devices and more particularly, to a modular system for simultaneous femoral fixation and femoral head resurfacing or replacement.

Fractures of the femoral shaft are among the most common fractures encountered. Subtrochanteric and femoral shaft fractures have been treated with the help of intramedullary nails inserted into the medullary canal of the femur to immobilize the fractured parts. Medullary fixation of fractures was introduced after World War II. A variety of medullary devices are now available. The most common are standard intramedullary nails wherein the largest diameter nail possible is inserted in the medullary canal to rigidly control forces exerted against the femur; interlocking intramedullary nails wherein transverse transfixing screws are inserted through standard medullary nails to control major proximal and distal fragments; and flexible intramedullary nails wherein fixation is achieved by three-point pressure from the insertion of a prebent flexible nail into a straight medullary canal.

The Grosse-Kempf nail manufactured by Howmedica Company of Rutherford, New Jersey is believed to be one of the earliest interlocking intramedullary nailing devices introduced into the United States. The Grosse-Kempf nail includes a threaded hole in the intramedullary rod for receiving the interlocking screw. The axis of the threaded hole coincides with a line between the greater to lesser trochanter and not in the direction of the femoral neck. European Patent No. 0 118 778 to Howmedica International, Inc. is illustrative to this type of nail.

Zickel U. S. Patent No. 3,433,220, which issued on March 18, 1969, discloses an intramedullary rod and cross-nail assembly which is useful in treating fractures occurring in the upper one-third or subtrochanteric portion of the femur. The Zickel nail is a solid intramedullary nail having a single proximal tri-flange cross-nail which is inserted in the direction of the femoral head. The cross-nail is locked into position by a set screw to prevent backing out.

The commercially available Kuntscher Y-nail includes a flanged cloverleaf shaped intramedullary nail which is inserted through a hole in a single femoral neck nail. The rod includes a longitudinal slit. The Kuntscher device is indicated only for unstable trochanteric fractures. Neither the Kuntscher device, nor the Zickel nail, includes distal anchoring means. The femoral neck nail of the Kuntscher device, which is angled toward the femoral neck, is locked into place by the intramedullary rod.

The Russell-Taylor interlocking nail system manufactured by Richards Medical Company of Memphis, Tennessee includes an intramedullary rod having two pairs of coaxial holes through its proximal end. The axes of the pairs of holes intersect to provide a left or right orientation for insertion of a single locking screw. The screw is designed to pass from the greater to the lesser trochanter. There is not sufficient mechanical support to allow usage of the locking screw in the direction towards the femoral head because the second pair of coaxial holes weaken the nail when loaded in that direction.

The techniques employed for using many of the aforementioned devices are set forth in CAMPBELL'S OPERATIVE ORTHOPAEDICS (Crenshaw 7th ed. 1987) pp. 1680-1712.

Total hip arthroplasty is currently the most common adult reconstructive hip procedure performed in the United States. Resurfacing arthroplasty is currently performed on a much smaller scale only on a select group of patients.

There are a variety of femoral head prostheses. Thompson and Moore developed a metallic femoral head prosthesis with medullary stems, called an endoprosthesis. Due to the potential for erosion of the bone on the pelvic side, and acetabulum is often resurfaced as well. A variety of acetabular cups are available. Resurfacing of the acetabulum is not always indicated, however. Various synthetic and ceramic femoral heads and cups provide low friction and good wear characteristics. Modular systems are available that provide a large number of combinations of head size and neck length and materials variations. The development of and techniques employed in arthroplasty of the hip are set forth in CAMPBELL'S OPERATIVE ORTHOPAEDICS, supra at pp. 1213-1491.

Baumann et al. U.S. Patent No. 4,101,985 discloses a hip-joint prosthesis having a ball head connected by a stem to an elongate, curved shaft for insertion into the medullary cavity. A screw extends through the femur into a bore in the shaft, stem and ball head to secure the prosthesis to the femur. The Baumann prosthesis is a one piece, total hip prosthesis with a lateral stabilizing screw.

In patients having total hip prostheses and a femoral shaft fracture or a fracture of the femoral neck/shaft that is likely to result in a vascular necrosis of the femoral head, long stem hip prostheses are currently used in treatment. With the current mode of treatment, however, shortening and rotation of the unstable shaft fracture can occur. In addition, hospitals must now stock a large number of sizes of long stem hip prostheses to accommodate patients' needs.

It is an object of the present invention to provide a modular system that offers the advantage of a total hip prosthesis, femoral head replacement or resurfacing with the superior fixation of an interlocking intramedullary nail.

It is a further object of the present invention to eliminate the necessity of stocking large numbers of sizes of long stem hip prostheses.

Finally, it is an object of the present invention to provide a modular system which facilitates removal of the device if the shaft fracture results in malunion or nonunion.

The aforementioned objects are satisfied by the combination of the present invention which provides an apparatus for implantation in the femur. The apparatus includes an intramedullary rod having a head portion and a stem portion for insertion into the femur, a separate prosthetic, generally spherical surface member for forming the exterior of the femoral head and in use being positioned in a confronting relationship to the acetabulum, and means for securing the rod to the prosthetic surface member.

The prosthetic surface member may take the form of a member for resurfacing the natural femoral head, a member for replacing the femoral head or a total hip prosthesis including a femoral head replacement and an acetabular cup. The head portion of the rod may have a first passage therethrough generally aligned in use with the centerline of the femoral head and neck. The securing means may be a screw which extends through the first passage of the head portion along the center line. Alternatively, the securing means may be a partial prosthesis attachment configured for cooperation with the rod and the prosthetic surface member. A second securing means may be provided for extension through a second passage in the head portion of the rod to prevent rotation.

The intramedullary rod may be any suitable length. In the longer rods, those extending generally the full length of the medullary canal, one or more distal interlocking means may be provided for use in treating comminuted or distal shaft fractures.

The present invention can be better understood by reference to the drawings in which:

FIG. 1 is a side elevation view of a preferred embodiment of the combination of the present invention;

FIG. 2 is a sectional view of the embodiment of FIG. 1 showing two screws for securing the rod to the surface member;

FIG. 3 is a side elevation view of an alternative embodiment of the combination of the present invention;

FIG. 4 is a side elevation view of an alternative embodiment of the combination of the present invention;

FIG. 5 is a side elevation view of a partial prosthesis attachment in combination with an intramedullary rod and two screws;

FIG. 6 is a side elevation view of an alternative embodiment of the partial prosthesis attachment in combination with an alternative embodiment of the intramedullary rod;

FIG. 7 is a cross section view through the line VII-VII of the partial prosthesis attachment and intramedullary rod of FIG. 6;

FIG. 8 is a top plan view along the line VIII-VIII of FIG. 6;

FIG. 9 is a side elevation view of an alternative embodiment of the partial prosthesis attachment in combination with an intramedullary rod;

FIG. 10 is a cross section view through line X-X of the partial prosthesis attachment and rod of FIG. 9;

FIG. 11 is a top plan view along the line XI-XI of FIG. 9.

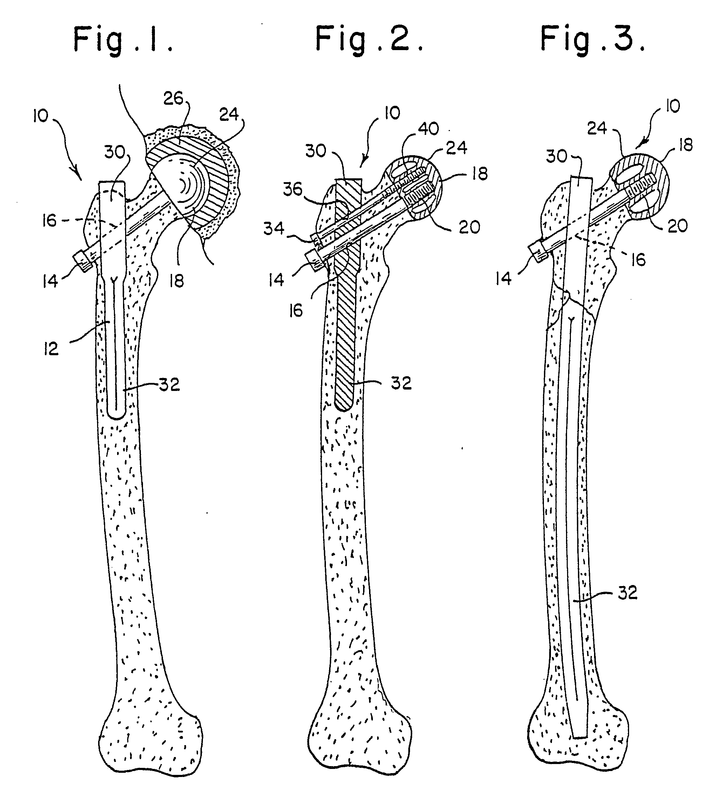

FIGS. 1-11 illustrate the preferred embodiments of the combination 10 of the present invention. Referring to FIG. 1, one embodiment of the combination includes an intramedullary nail or rod 12, a screw or bolt 14 which extends through a passage 16 and a prosthetic surface member 18 in the position of the natural femoral head. The screw 14, as shown in FIGS. 2 and 3, is threaded within a threaded bore 20 of the prosthetic surface member 18. As shown the screw has a smooth surface portion and a threaded portion. Alternatively, the screw may be threaded along its full length.

The prosthetic surface member 18 can take the form of a total hip prosthesis, as shown in FIG. 1. The total hip prosthesis includes a prosthetic femoral head 24 and an acetabular cup 26. The acetabular cup 26 is cemented in the prepared acetabulum of the pelvis by known procedures as set forth in CAMPBELL'S OPERATIVE ORTHOPAEDICS, supra.

In some applications, an acetabular cup 26 is not used. In those instances, the prosthetic femoral head rotates within the natural hip socket in confronting relationship with the acetabulum.

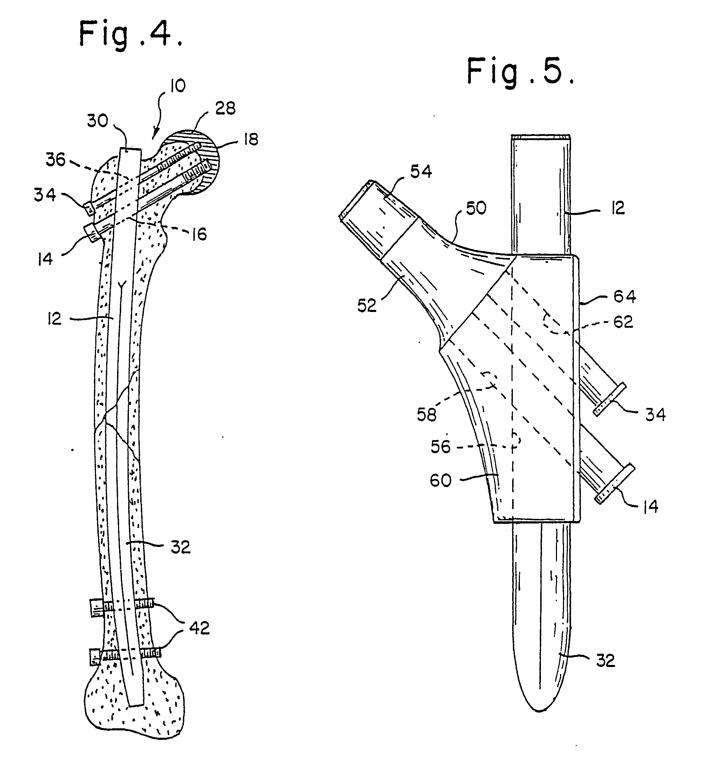

Alternatively, the prosthetic surface member may be in the form of a resurfacing head 28 on a prepared natural femoral head as shown in FIG. 4. The screw 14 is threaded into the head 28 to secure the head 28 to the rod 12.

"Prepared" as used herein with respect to the prepared acetabulum and the prepared natural femoral head means an acetabulum or femoral head that has been surgically altered in preparation for attachment of the prosthetic acetabular cup 26 or resurfacing femoral head 28.

The rod 12 has a head portion 30 and a stem portion 32. The head portion 30 may include at least one passage, such as passage 16 which, when the rod 12 is placed in the medullary canal of the femur to the desired depth, is aligned generally with the centerline of the femoral head and neck. The head portion 30 of rod 12 may, alternatively, have no passages therethrough or may include two such passages, passage 16 and a second passage 36 being generally parallel to and smaller in diameter than the first passage 16. A second screw 34 extends through the second passage 36 to prevent rotation. The second screw 34, like the first screw 14, secures the rod 12 to the prosthetic surface member 18.

The various embodiments of the prosthetic surface member 18, prosthetic femoral head 24 or resurfacing head 28 are adapted to engage either one or two screws. The prosthetic femoral head 24 shown in FIG. 3, for example, includes a single bore 20 for engaging screw 14. The embodiment of prosthetic femoral head 24 shown in FIG. 2, however, has, in addition to bore 20, a second bore 40 for engaging the second screw 34. The embodiment of resurfacing head 28 shown in FIG. 4 engages both screws 14 and 34. An alternative embodiment (not shown) may engage only one screw.

The rod 12 may be any suitable length. Long and short varieties are shown in the Figures. One or more distal interlocking screws 42 (two are shown) may be provided for insertion through the stem portion 32 of rod 12. Distal interlocking screws are indicated in fixation of comminuted or distal shaft fractures. Two distal screws 42 prevent what is known as toggle.

The prosthetic surface member 18 is readily detachable from the rod 12 by removal of the screw 14 and, when present, screw 34.

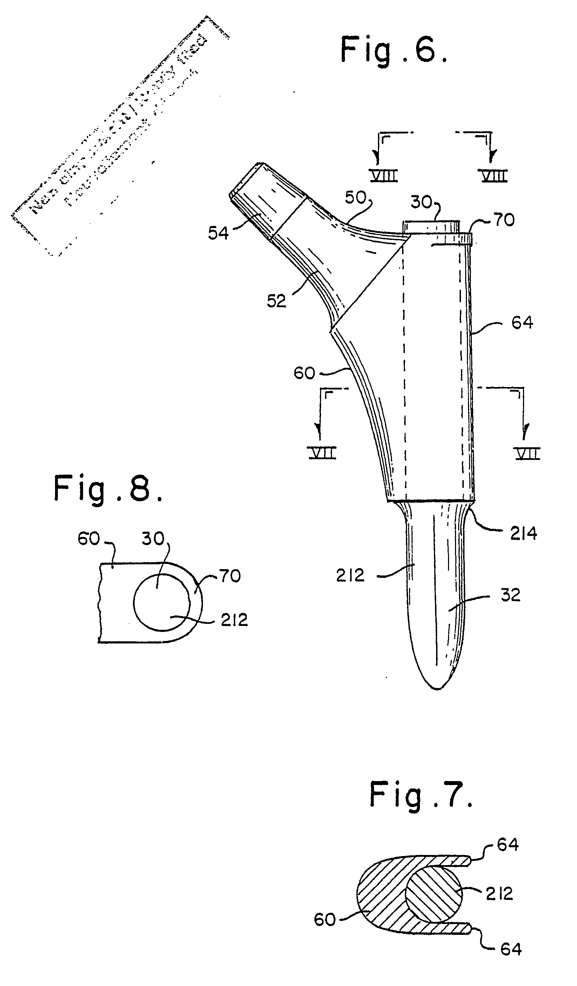

FIGS. 6-8 illustrates a partial prosthesis attachment 50 for use in combination with an intramedullary rod 212 and a prosthetic femoral head similar to the head 24 shown in FIG. 1. The partial prosthesis attachment 50 is configured for insertion into the proximal portion of the femur and functions as a means for securing rod 212 to the prosthetic femoral head 24. A shoulder 214 is provided on the stem portion 32 of rod 212 to prevent the partial prosthesis attachment 50 from subsiding. The section 52 of the attachment 50, when inserted, extends into the femoral neck. A prosthetic femoral head or a bipolar head would be attached by suitable known means to the end 54 of section 52 when in use. As shown in FIG. 7, edge 64 of attachment 50 is curved to provide a cradle for rod 212 to rest against. In this embodiment, there is no need for passages through the rod as are provided in rod 12. A ring portion 70, as shown in FIGS. 6 and 8 acts as a support for the partial prosthesis attachment 50.

As shown in FIG. 5, rod 12 may alternatively extend through a circular longitudinal bore 56 in section 60 of attachment 50. The alternative embodiment shown in FIG. 5 may also include transverse passages 58 and 62, respectively, in section 60 of attachment 50 for use with the embodiment of rod 12 having passages 16 and 36. The passages 58 and 62 align with passages 16 and 36 in rod 12. Screws 14 and 34 extend through the passages 58, 62, 16 and 36. Although two passages 58 and 62 and two screws 14 and 34 are shown, those skilled in the art will appreciate that only one passage and one screw may be employed to secure the attachment 50 to rod 12.

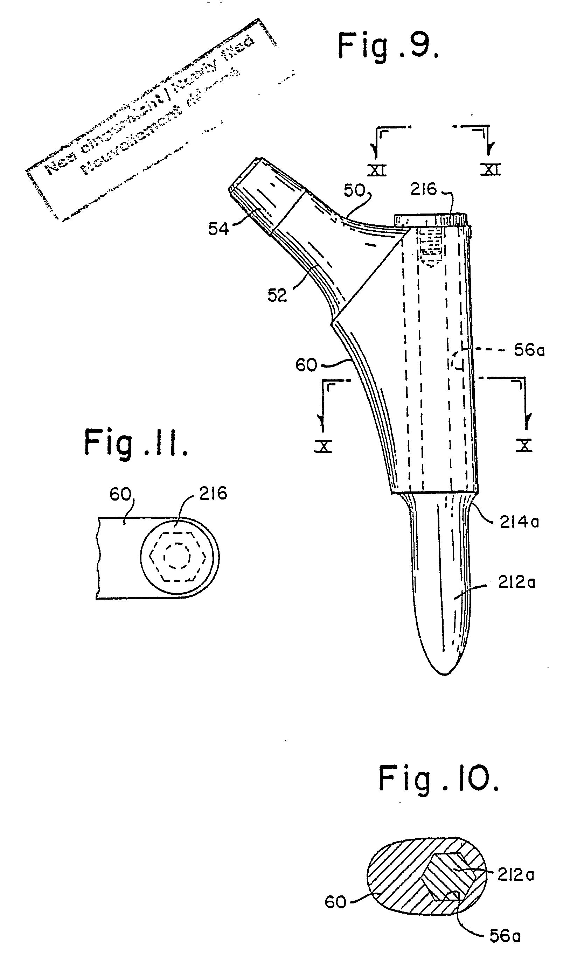

FIGS. 9-11 illustrate an embodiment of the partial prosthesis attachment 50 having a hexagonal bore 56a. A hexagonal rod 212a having a shoulder 214a is received in bore 56a. The shape prevents rotation of the rod 212a within bore 56a. Those skilled in the art will recognize that other shapes may be employed to prevent rotation of rod 12 or 212 within bore 56. A cap 216 screws into a counterbore in the head portion of rod 212a and together with shoulder 214a, secures the rod 212 and partial prosthesis attachment 50 together as one unit.

The combination 10 of the present invention provides a modular system for handling a variety of femoral fractures in combination with hip and femoral head involvement. The invention offers the advantages of a total hip prosthesis combined with the superior fixation of an interlocking intramedullary nail.

## Claims

1. Apparatus for implantation in a femur comprising:
an intramedullary rod having a head portion and a stem portion for insertion into the femur;
a separate prosthetic generally spherical surface member for forming the exterior of the femoral head and in use being positioned in a confronting relationship to the acetabulum; and
means for securing said rod to said prosthetic surface member.

2. The apparatus recited in claim 1 wherein said head portion of said rod has a first passage therethrough generally aligned in use with the centerline of the femoral head and neck, and said securing means extends through said first passage of said head portion of said rod along said centerline.

3. The apparatus recited in claim 2 wherein said head portion of said rod has a second passage therethrough generally parallel to said first passage and further comprising a second means extending through said second passage of said head portion of said rod for securing said rod to said prosthetic surface member.

4. The apparatus recited in claim 1 wherein said prosthetic surface member is a prosthetic member for replacement of the femoral head.

5. The apparatus recited in claim 1 wherein said securing means comprises a partial prosthetic attachment for insertion in the proximal portion of the femur, said attachment having a main body portion configured for cooperation with said rod and an extension from said main body portion, said extension extending in use into the femoral neck and being configured for attachment to said prosthetic surface member for replacement of the femoral head.

6. The apparatus recited in claim 5 wherein said rod has a first passage therethrough generally aligned in use with the centerline of the femoral head and neck and said main body portion has a passage therethrough for alignment in use with said first passage of said rod, and a second securing means for insertion through said passage of said main body portion and said first passage.

7. The apparatus recited in claim 1 further comprising an acetabular cup for resurfacing the acetabulum.

8. The apparatus recited in claim 1 further comprising at least one passage in said stem portion of said rod and interlocking means for extension through said at least one passage in said stem portion of said rod.

9. The apparatus recited in claim 1 wherein said prosthetic surface member is a total hip prosthesis.

27·02·90

New eingereicht / Newly filed
Nouvellement déposé

# Fig.1.

# Fig.2.

# Fig.3.

# Fig.4.

# Fig.5.

# Fig.6.

# Fig.8.

# Fig.7.

# Fig .9.

50

216

54

52

56a

60

214a

212a

# Fig .11.

60

216

# Fig .10.

212a

60

56a

European Patent Office

**EUROPEAN SEARCH REPORT**

Application Number

EP 90 30 0992

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 010 527 (LIMA) <br> * Entire document * | 1,2,4,5 ,7-9 | A 61 F 2/36 |
| Y | | 3,6 | |
| Y | US-A-4 733 654 (MARINO) <br> * Figure 1; column 2, lines 31-67 * | 3,6 | |
| A | DE-A-2 854 334 (ERLER) | | |
| A | FR-A-2 406 433 (OH) | | |
| A | EP-A-0 257 359 (BOEHRINGER MANNHEIM CORP.) | | |

TECHNICAL FIELDS SEARCHED (Int. Cl.5)

A 61 F

The present search report has been drawn up for all claims

| Place of search | Date of completion of the search | Examiner |
|---|---|---|
| THE HAGUE | 10-05-1990 | STEENBAKKER J. |

EPO FORM 1503 03.82 (P0401)